Europäisches Patentamt

European Patent Office

Office européen des brevets

(19)

(11) Veröffentlichungsnummer: **0 101 083**

**A1**

(12) # EUROPÄISCHE PATENTANMELDUNG

(21) Anmeldenummer: 83108009.8

(22) Anmeldetag: 12.08.83

(51) Int. Cl.³: **A 62 D 3/00**
**A 61 L 9/14, A 61 L 9/01**

(30) Priorität: 12.08.82 DE 3230029

(43) Veröffentlichungstag der Anmeldung:
22.02.84 Patentblatt 84/8

(84) Benannte Vertragsstaaten:
AT BE CH DE FR GB IT LI LU NL SE

(71) Anmelder: Grunert, Wilhelm O. H.
Ringstrasse 6
D-8033 Krailling(DE)

(72) Erfinder: Grunert, Wilhelm O. H.
Ringstrasse 6
D-8033 Krailling(DE)

(74) Vertreter: Dr. Elisabeth Jung Dr. Jürgen Schirdewahn
Dipl.-Ing. Claus Gernhardt
P.O. Box 40 14 68 Clemensstrasse 30
D-8000 München 40(DE)

(54) Verfahren zur Neutralisierung von Reizstoffen und giftigen Gasen und zur Durchführung des Verfahrens geeignete Präparate.

(57) Die Neutralisierung von Reizstoffen und giftigen Gasen, wie Ammoniak, Rauch- und Brandgase, Kohlenmonoxid, wird ermöglicht durch Behandeln der verseuchten Gegenstände und Personen bzw. der verseuchten Atmosphäre mit einer Lösung, die Polyvinylpyrrolidon enthält. Diese Lösung wird zweckmäßig aufgesprüht bzw. in der Atmosphäre nebelförmig verteilt. Ein zur Anwendung in diesem Verfahren besonders geeignetes Präparat enthält in wässriger Lösung mindestens 0,1 Gewichtsprozent Polyvinylpyrrolidon, gegebenenfalls zusammen mit Hilfs- oder Trägerstoffen.

Anstelle einer den Wirkstoff enthaltenden Lösung kann dieser auch in Form feiner Teilchen angewendet werden, welche in fester Form z.B. in der Atmosphäre versprüht werden.

**ELISABETH JUNG** DR. PHIL., DIPL.-CHEM.
**JÜRGEN SCHIRDEWAHN** DR. RER. NAT., DIPL.-PHYS.
**CLAUS GERNHARDT** DIPL.-ING.

PATENTANWÄLTE
EUROPEAN PATENT ATTORNEYS

8000 MÜNCHEN 40 **0101083**
P. O. BOX 40 14 68
CLEMENSSTRASSE 30
TELEFON: (089) 34 50 67
TELEGRAMM/CABLE: INVENT MÜNCHEN
TELEX: 5-29 686
TELECOPIERER (FAX): (089) 39 92 39 (GR. II/III)

12. AUG. 1983

S 469 C

Wilhelm O.H. Grunert
Krailling bei München

---

Verfahren zur Neutralisierung von Reizstoffen und giftigen
Gasen und zur Durchführung des Verfahrens geeignete Präparate

---

Die menschliche Umwelt wird in der heutigen hochindustrialisierten Gesellschaft häufig durch Reizstoffe und giftige Gase
gefährdet, welche teilweise aufgrund von Unachtsamkeit oder Unfällen und teilweise aufgrund bewußter Aktionen in die Atmosphäre gelangen.

Beispiele hierfür sind die bei Bränden gebildeten Rauch- und
Brandgase. So entwickelt sich beim Brand von Kunstdünger eine
stark ammoniakhaltige Atmosphäre, während bei Bränden von
kunststoffhaltigen Materialien öfters chlor- bzw. salzsäurehaltige Dämpfe gebildet werden.

Bei Explosionen in chemischen Fabriken können ganze Stadtteile
durch Schleimhaut reizende Chlorgase verseucht werden und auch
beim Transport von Chemikalien werden infolge von Verkehrsunfällen immer wieder Reizstoffe und giftige Gase freigesetzt,
welche Hilfs- und Rettungsarbeiten der Polizei und der technischen Notdienste erschweren oder sogar unmöglich machen.

Auch in Laboratorien können Unglücksfälle dazu führen, daß
Reiz- und Giftstoffe, wie Ammoniak, Formaldehyd, Schwefeldioxid und Schwefelwasserstoff, in die Arbeitsräume emittiert werden und die dort arbeitenden Personen gefährden.

Es wäre daher außerordentlich erwünscht, die Wirkung derartiger Reizstoffe und giftigen Gase auf einfache Weise möglichst schnell zu neutralisieren und dadurch die Gefahr für die betroffenen Personen und die menschliche Umwelt zu verringern.

Überraschenderweise hat sich gezeigt, daß dieses technische Problem dadurch gelöst werden kann, daß man die verseuchten Gegenstände bzw. die verseuchte Atmosphäre mit einem fein verteilten eiweißartigen Wirkstoff behandelt.

Das erfindungsgemäße Verfahren zur Neutralisierung von Reizstoffen und giftigen Gasen ist daher dadurch gekennzeichnet, daß man die verseuchten Gegenstände und Personen bzw. die verseuchte Atmosphäre mit einem
wasser- und alkohollöslichen Polyvinylpyrrolidon in fein verteilter Form behandelt.

Der hier verwendete Ausdruck "alkohollöslich" bezieht sich auf die Löslichkeit in niedrigen aliphatischen Alkoholen mit bis zu 3 Kohlenstoffatomen im Molekül.

Vorzugsweise wird im Rahmen des erfindungsgemäßen Verfahrens eine wässrige Lösung zu einem Nebel versprüht, insbesondere wenn abgeschlossene Räume entgiftet werden müssen. Hierfür eignen sich z.B. mit und ohne Treibmittelzusatz arbeitende Sprühdosen, ferner Handsprühgeräte und sogenannte Nebelkeulen. Die Lösung kann aber auch mit Bürsten, Lappen oder anderen Geräten als solche auf verseuchte Gegenstände oder Personen aufgebracht werden, wodurch ein weiteres Vergasen der häufig in Flüssigform vorliegenden Reizstoffe verhindert wird und gleichzeitig eine Neutralisierung der Reiz- bzw. Giftwirkung erfolgt.

Es ist aber auch möglich, das Polyvinylpyrrolidon in fester
Form zur Anwendung zu bringen, indem man ein feines Pulver versprüht, auf dem sich z.B. in einem Raum befindliche nebelförmige Reizstoffe niederschlagen und dadurch neutralisiert werden.

Für dieses Neutralisierungsverfahren verwendbare Polyvinylpyrrolidone weisen günstigerweise ein mittleres Molekulargewicht
im Bereich von 5000 bis 2 000 000 auf. Solche Polyvinylpyrrolidone sind für andere Zwecke im Handel erhältlich.

Die Erfindung bezieht sich auch auf Präparate, welche sich zur
Durchführung dieses neuen Verfahrens eignen.

                                             vorteilhaft
Diese Präparate enthalten das Polyvinylpyrrolidon/in Form
einer Lösung, gegebenenfalls in Kombination mit Konservierungsmitteln sowie anderen Hilfs- und Trägerstoffen. Besonders
zweckmäßig sind Präparate, welche Wasser als Lösungsmittel enthalten, gegebenenfalls unter Mitverwendung von Dimethyläther.

                          solche
Als Lösungsmittel für  /  erfindungsgemäße Präparate eignen
sich außer Wasser auch einwertige bzw. mehrwertige Alkohole
                              mit Wasser
mit bis zu 6 Kohlenstoffatomen. Zusammen / können gewünschtenfalls bis zu 50 Gewichtsprozent, vorzugsweise bis zu 30 Gewichtsprozent und insbesondere bis zu 10 Gewichtsprozent 2- bis
6-wertige Alkohole mit bis zu 6 Kohlenstoffatomen im Molekül
verwendet werden. Beispiele für solche Alkohole sind Methanol,
Äthanol, Propanol, Isopropanol, Butanol, sek.-Butanol, Amylalkohol, 2-Äthyl-1-hexanol, Cyclohexanol, Äthylenglykol, Propylenglykol, 1,3-Butandiol, 1,4-Butandiol und Glycerin. Auch
Ketonalkohole, wie z.B. Diacetonalkohol, können als Lösungsmittel eingesetzt werden.

Ferner eignen sich als Lösungsmittel aliphatische Carbonsäuren.
wie z.B. Ameisensäure, Essigsäure und Propionsäure. Auch Ätheralkohole, wie Glykoläther, Diäthylenglykol, Triäthylenglykol, Hexamethylenglykol, Polymethylenglykol 400 und 2,2'-Thiodiäthanol sind geeignet. Weitere geeignete Lösungsmittel sind Laktone, wie Butyrolakton; Ester, wie Äthyllaktat; Ketone, wie Methylcyclohexanon; chlorierte Kohlenwasserstoffe, wie z.B. Methylendichlorid, Chloroform, Äthylendichlorid; ferner Laktame, wie 2-Pyrrolidon, N-Methyl-2-pyrrolidon und N-Vinyl-2-pyrrolidon; sowie Amine, z.B. Butylamin, Cyclohexylamin, Anilin, Äthylendiamin, Pyridin, Morpholin, 2-Aminoäthanol, Diäthanolamin, Triäthanolamin, Aminoäthyläthanolamin, 2-Hydroxyäthylmorpholin, 2-Amino-2-methyl-1-propanol; und Nitroparaffine, wie z.B. Nitromethan und Nitroäthan.

Weiterhin können die folgenden halogenierten Kohlenwasserstoffe auch in Kombination mit niedrigen aliphatischen Alkoholen mit 1 bis 4 und vorzugsweise 2 bis 3 Kohlenstoffatomen im Molekül eingesetzt werden: Trichlorfluormethan, Dichlordifluormethan, 1,1,2-Trichlor-1,2,2-trifluoräthan, 1,2-Dichlor-1,1,2,2-tetrafluoräthan, 1-Chlor-1,1-difluoräthan. Das Mischungsverhältnis von Kohlenwasserstoff zu Alkohol liegt im allgemeinen im Bereich von 90 : 10 bis 60 : 40 Gewichtsteilen, vorzugsweise im Bereich von 80 : 20 bis 70 : 30 Gewichtsteilen.

Die für das erfindungsgemäße Verfahren einsetzbaren Präparate enthalten das Polyvinylpyrrolidon üblicherweise in einer Menge von mindestens 0,1 Gewichtsprozent. Vorzugsweise beträgt die Polyvinylpyrrolidon-Konzentration 0,2 bis 10 Gewichtsprozent und insbesondere 0,3 bis 3 Gewichtsprozent, jeweils bezogen auf das Gesamtgewicht der Lösung.

In Sprühpräparaten liegt das Polyvinylpyrrolidon im allgemeinen in Mengen von 0,3 bis 5 Gewichtsprozent, vorzugsweise in Mengen von 0,5 bis 3 Gewichtsprozent und insbesondere in Mengen von etwa 1 bis 2 Gewichtsprozent vor, bezogen auf das Gesamtgewicht des Sprühpräparates abzüglich des Treibmittels.

Für pulverförmige Präparate soll das Polyvinylpyrrolidon möglichst feinteilig sein. Es kann in diesen Präparaten zusammen mit üblichen Hilfs- und Trägerstoffen vorliegen.

Solche Präparate können auch zusammen mit Treibmitteln konfektioniert werden und eignen sich dann als Sprühpräparate.

Hilfsstoffe, welche den erfindungsgemäßen Präparaten zugesetzt werden können, sind z.B. Tenside und Konservierungsmittel, wie Kaliumsorbat.

Für bestimmte Anwendungszwecke können die festen oder flüssigen Präparate der Erfindung auch Desinfektionsmittel enthalten.

Die erfindungsgemäßen Präparate eignen sich für den Großeinsatz bei der Feuerwehr, bei den technischen Hilfs- bzw. Notdiensten und bei der Polizei, da sie an Ort und Stelle aus Konzentraten herstellbar sind und sich als völlig unschädlich für Mensch und Tier erwiesen haben. Infolge des Fehlens jeglichen Eigengeruchs beim Polyvinylpyrrolidon haftet den behandelten Personen und Gegenständen auch kein störender Geruch an, so daß der Einsatz der Präparate unbedenklich erfolgen kann.

Die Beispiele erläutern die Erfindung.

B e i s p i e l 1

Durch Auflösen von 2 g Polyvinylpyrrolidon in 90 g Isopropylalkohol und Zusatz von 5 g Triäthylenglykol wird ein Spraypräparat hergestellt und zusammen mit 15 g Frigen 11/12 sowie 7,5g $CO_2$-Gas als Treibmittel in der üblichen Weise in Behälter verpackt.

a) In einem Laboratorium werden Ammoniak und Formaldehyd nacheinander getrennt versprüht, bis die an dem Versuch teilnehmenden Personen ernstlich unter der Reizung der Atemwege und der Augen leiden. Dann wird das vorstehend beschriebene Sprühpräparat angewendet und der Raum 3 Minuten sich selbst überlassen. Beim Wiederbetreten kann in der Luft kein Geruch nach Ammoniak und Formaldehyd mehr wahrgenommen werden, und es werden auch keine Reizungserscheinungen von den betreffenden Personen beobachtet.

b)  In einer geschlossenen Garage läßt man einen Motor laufen, so daß sich der Raum mit giftigen Abgasen füllt.
Bei laufendem Motor wird das Sprühpräparat angewendet.
Bei den in der Garage anwesenden Versuchspersonen zeigen sich keinerlei Vergiftungserscheinungen, wie Übelkeit oder Kopfschmerzen.

B e i s p i e l  2

Es wird ein Spray-Präparat der nachstehenden Zusammensetzung 2,5 Gewichtsprozent Polyvinylpyrrolidon
1,0 Gewichtsprozent Äthanol
1 Teil pro Million Benzalkoniumchlorid
32 Gewichtsprozent Dimethyläther
Rest entmineralisiertes Wasser verwendet.

Auf dem Boden eines Raumes wurde handelsübliche wäßrige Lösung von Kaliumhypochlorit(Eau de Javelle) verschüttet und
flächenmäßig verteilt, so daß die sich entwickelnden Chlordämpfe sich im ganzen Raum ausbreiteten.

Nach 30 Minuten wurde in der Atmosphäre des Raumes ein Präparat der vorstehenden Zusammensetzung sorgfältig versprüht
und gleichzeitig der Boden mit Wasser gereinigt, um eine
weitere Bildung von Chlordämpfen zu unterbinden. Durch das
Besprühen der Atmosphäre war die Reizwirkung im Raum soweit
neutralisiert worden, daß dieser Reinigungsvorgang überhaupt
vorgenommen werden konnte.

30 Minuten nach der ersten Sprühbehandlung war nur noch ein schwacher Chlorgeruch zu bemerken. Durch Wiederholung dieser Behandlung konnten die letzten Reizerscheinungen behoben werden. Die zweistündige Nachkontrolle in Abständen von 10 Minuten bestätigte den Erfolg der Neutralisierungsbehandlung.

B e i s p i e l 3

Im Freien wurden Teile von Autoreifen und Schwefelstreifen mit Benzin übergossen und dann in Brand gesetzt. Nach 10 Minuten war die Rauch- und Gasentwicklung so stark, daß ein Vordringen zum Brandherd durch die dabei auftretenden Reizerscheinungen praktisch nicht mehr möglich war.

Durch Versprühen eines Präparates entsprechend der Zusammensetzung von Beispiel 2 konnte in den Rauchschwaden eine Art Schneise geschaffen werden, welche es einer Versuchsperson gestattete, ohne Belästigung durch Husten- oder Tränenreizungen bzw. ohne Atembeschwerden zum Brandherd zu gelangen und den Brand mit Wasser zu löschen.

In der dabei gebildeten Dampf- und Rauchwolke konnte durch Einsatz des Sprühpräparates wiederum ein passierbarer Korridor geschaffen werden, durch den im Ernstfall Rettungsmannschaften zur Bergung von Menschen zum Brandherd gelangen könnten. Auch in diesem Korridor wurden keine Reizwirkungen bei Mensch und Tier (Haushund) beobachtet.

B e i s p i e l 4

In einer Fleischwarenfabrik wurde Nitritpökelsalz mit Wasser zur Naßpökelung von Kochschinken angesetzt.

Zur Verbesserung und länger anhaltenden Stabilität der Fleischfarbe wurde als Antioxydans eine kleine Menge Ascorbinsäure beigegeben. Durch diese Beimischung entstand eine starke gasartige Reaktion, welche beim Personal Übelkeit und Kopfweh bis zur Bewußtlosigkeit auslöste.

In diesem Falle wurde die gasartige Reaktion bei der Entstehung durch Versprühung eines erfindungsgemäßen Präparats (über der Oberfläche der Lake und dem umgebenden Raum) sofort neutralisiert. Auf diese Weise konnte der notwendige Arbeitsablauf ohne weitere gesundheitliche Störungen und Vergiftungserscheinungen beim Personal fortgesetzt werden.

B e i s p i e l  5

In der heißen Jahreszeit entstehen öfters Schwierigkeiten in Kläranlagen dadurch, daß im Abwasserschlamm eine übermäßige Bildung von Faulgas einsetzt.

Es wurde daher zum Abwasserschlamm eine wässrige Lösung von Polyvinylpyrrolidon zugesetzt und außerdem wurde die Oberfläche des Schlammsammelbeckens mit einem Präparat entsprechend der Zusammensetzung von Beispiel 2 besprüht.

Trotz anhaltender Sommerhitze hatte die Neutralisierungsbehandlung eine sich über etwa eine Woche erstreckende Wirkung.

B e i s p i e l  6

Beim Sprengen von Dolomitgestein und beim Vermahlen desselben zu Dolomitverputz entstehen feinste lungengängige Staubwolken, welche bei den beschäftigten Personen Silicose verursachen und außerdem zu Reizerscheinungen bei den Schleimhäuten führen (Silicosehusten).

Vor Ort wurden in einem Steinbruch die bei Sprengungen bzw. bei der Gesteinsmahlung gebildeten Staubwolken intensiv mit einem erfindungsgemäßen Präparat (Zusammensetzung gemäß Beispiel 2 ) in Nebelform behandelt. Die Staubteilchen fielen sofort zu Boden und die Reizerscheinungen bei den anwesenden Personen hörten auf.

0101083

Patentansprüche

1. Verfahren zur Neutralisierung von Reizstoffen und giftigen Gasen, d a d u r c h g e k e n n z e i c h n e t , daß man die verseuchten Gegenstände und Personen bzw. die verseuchte Atmosphäre mit einem wasser- und alkohollöslichen Polyvinylpyrrolidon in fein verteilter Form behandelt.

2. Verfahren nach Anspruch 1, dadurch gekennzeichnet, daß man das Polyvinylpyrrolidon in Form fester Teilchen anwendet.

3. Verfahren nach Anspruch 1, dadurch gekennzeichnet, daß man das Polyvinylpyrrolidon in Form einer wässrigen oder alkoholischen Lösung anwendet.

4. Verfahren nach Anspruch 1 und 3, dadurch gekennzeichnet, daß man eine wässrige Lösung verwendet, die zusätzlich Konservierungsmittel enthält.

5. Verfahren nach Anspruch 1, 3 und 4, dadurch gekennzeichnet, daß man eine wässrige Lösung verwendet, die weitere Hilfs- und/oder Trägerstoffe enthält.

6. Verfahren nach Anspruch 5, dadurch gekennzeichnet, daß man eine wässrige Lösung verwendet, die Dimethyläther enthält.

7. Verfahren nach Anspruch 1 und 3, dadurch gekennzeichnet, daß man eine Lösung von Polyvinylpyrrolidon in Isopropylalkohol verwendet.

8. Verfahren nach Anspruch 1 bis 7, dadurch gekennzeichnet, daß man das Polyvinylpyrrolidon in Form fester Teilchen bzw. die Behandlungslösung auf die verseuchten Personen oder Gegenstände aufsprüht.

9. Verfahren nach Anspruch 1 bis 8, dadurch gekennzeichnet, daß man die verseuchte Atmosphäre mittels der Polyvinylpyrrolidonlösung in Nebelform behandelt.

10. Verfahren nach Anspruch 1 bis 7, dadurch gekennzeichnet, daß man die Behandlungslösung mittels Bürsten, Lappen oder anderen Geräten auf die Oberfläche von verseuchten Gegenständen oder Personen aufbringt.

11. Präparat zur Durchführung des Verfahrens nach Anspruch 1 in Form einer Lösung von wasser- und alkohollöslichem Polyvinylpyrrolidon.

12. Präparat nach Anspruch 11, dadurch gekennzeichnet, daß die Lösung mindestens 0,1 Prozent Polyvinylpyrrolidon enthält.

13. Präparat nach Anspruch 11 und 12, dadurch gekennzeichnet, daß die Polyvinylpyrrolidonkonzentration im Bereich von etwa 0,2 bis 10 % liegt.

14. Präparat nach Anspruch 11 bis 13, dadurch gekennzeichnet, daß die Polyvinylpyrrolidonkonzentration im Bereich von etwa 0,3 bis 3 % liegt.

15. Präparat nach Anspruch 11 bis 14, dadurch gekennzeichnet, daß das Polyvinylpyrrolidon ein Molekulargewicht im Bereich von 5000 bis etwa 2 000 000 aufweist.

16. Präparat nach Anspruch 15, dadurch gekennzeichnet, daß das Polyvinylpyrrolidon ein Molekulargewicht im Bereich von 10 000 bis etwa 1 000 000 aufweist.

17.    Präparat nach Anspruch 16, dadurch gekennzeichnet, daß das Polyvinylpyrrolidon ein Molekulargewicht im Bereich von etwa 13 000 bis etwa 45 000 aufweist.

18.    Präparat nach Anspruch 11 bis 17, dadurch gekennzeichnet, daß das Lösungsmittel Wasser und/oder ein einwertiger oder mehrwertiger Alkohol mit bis zu 6 Kohlenstoffatomen im Molekül oder ein Gemisch aus Wasser mit einwertigen oder mehrwertigen Alkoholen mit bis zu 6 Kohlenstoffatomen im Molekül ist.

19.    Präparat nach Anspruch 18, dadurch gekennzeichnet, daß das Lösungsmittel Methanol, Äthanol, Propanol, Isopropanol, Butanol, sek.-Butanol, Amylalkohol, 2-Äthyl-1-hexanol, Cyclohexanol, Phenol (50°C), Äthylenglykol, Propylenglykol, 1,3-Butandiol, 1,4-Butandiol und/oder Glycerin ist oder einen der vorgenannten Stoffe enthält.

20.    Präparat nach Anspruch 11 bis 17, dadurch gekennzeichnet, daß das Lösungsmittel ein Ketonalkohol, eine aliphatische Carbonsäure, ein Glykoläther, ein Lakton, ein Ester, ein chlorierter Kohlenwasserstoff, ein Laktam, ein Amin oder ein Nitroparaffin ist oder einen der vorgenannten Stoffe enthält.

21.    Präparat nach Anspruch 20, dadurch gekennzeichnet, daß das Lösungsmittel Diacetonalkohol, Ameisensäure, Essigsäure, Propionsäure, Diäthylenglykol, Triäthylenglykol, Hexamethylenglykol, Polyäthylenglykol 400, 2,2'-Thiodiäthanol, Butyrolakton, Methylcyclohexanon, Methylendichlorid, Chloroform, Äthylendichlorid, 2-Pyrrolidon, N-Methyl-2-pyrrolidon, N-Vinyl-2-pyrrolidon, Butylamin, Cyclohexylamin, Anilin, Äthylendiamin, Pyridin, Morpholin, 2-Aminoäthanol, Diäthanolamin, Triäthanolamin, Aminoäthyläthanolamin, 2-Hydroxyäthylmorpholin, 2-Amino-2-methyl-1-propanol, Nitromethan und/oder Nitroäthan ist oder einen der vorgenannten Stoffe enthält.

22.    Präparat zur Durchführung des Verfahrens nach Anspruch 1, enthaltend wasser- und alkohollösliches Polyvinylpyrrolidon in Pulverform, gegebenenfalls zusammen mit üblichen Hilfs- und Trägerstoffen.

23.    Sprühpräparat, enthaltend ein Präparat nach Anspruch 1$_1$ bis 22 in Mischung mit einem Treibmittel.

24.    Sprühpräparat, enthaltend ein Präparat nach Anspruch 11 bis 22 in Mischung mit Dimethyläther.

0101083

Europäisches
Patentamt

**EUROPÄISCHER RECHERCHENBERICHT**

Nummer der Anmeldung

EP 83 10 8009

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | DE-B-1 767 851 (KERNFORSCHUNGSANLAGE JÜLICH) <br><br> * Insgesamt * <br><br> --- | 1,3,7, 9,11- 13,18, 19,23 | A 62 D 3/00 <br> A 61 L 9/14 <br> A 61 L 9/01 |
| X | DE-A-2 557 365 (R. KUERNER) <br><br> * Seite 4, Zeile 24 - Seite 6, Zeile 16; Seite 8, Zeilen 12-19 * | 1,3,4, 7,9,11 -21,23 | |
| A | - <br><br> * Beispiele 3,10,11; Ansprüche * <br><br> --- | 5,10, 22 | |
| A | GB-A-2 050 388 (I.C.I.) <br><br> * Seite 2, Zeilen 41-57; Ansprüche * <br><br> ----- | 1,2,8, 22 | RECHERCHIERTE SACHGEBIETE (Int. Cl. ³) <br><br> A 62 D <br> A 61 L |

Der vorliegende Recherchenbericht wurde für alle Patentansprüche erstellt.

| Recherchenort <br> DEN HAAG | Abschlußdatum der Recherche <br> 11-11-1983 | Prüfer <br> FLETCHER A.S. |
|---|---|---|